# EUROPEAN PATENT APPLICATION

(11) **EP 3 278 837 A1**
(43) Date of publication of application: **07.02.2018**
(21) Application number: 16183085.6
(22) Date of filing: 05.08.2016
(51) Int. Cl.: A61N 1/39, A61B 5/0205, A61H 31/00, G08B 21/06

(54) **SYSTEM FOR AUTOMATIZATION OF LIFE-PRESERVING MEASURES IN A CAR**

(71) Applicant: Technische Hochschule Ingolstadt, 85049 Ingolstadt (DE)
(72) Inventor: Hasirlioglu, Sinan, 85055 Ingolstadt (DE); Doric, Igor, 83301 Traunreut (DE); Brandmeier, Thomas, 93173 Wenzenbach Irlbach (DE)
(74) Representative: Lucke, Andreas

(57) **Abstract**

A system (10) for automatically applying life-preserving measures to a passenger in a car suffering a cardiorespiratory arrest, wherein the system comprises: a sensing module (40) configured for receiving sensing signals from one or more sensors (42), said sensors (42) configured for obtaining said sensing signals related to vital signs of the passenger, said sensing signals allowing the sensing module (40) to detect when the passenger is suffering a cardiorespiratory arrest; a driving module (30) configured for driving a tensioning movement of a seat belt tensioning mechanism (32) so as to apply cardiopulmonary resuscitation to a passenger secured by the seat belt by compressing his/her chest; and a control unit (20) operatively connected to the sensing module (40) and the driving module (30), wherein the control unit (20) is configured to control the driving module (30) to operate the seat belt tensioning mechanism (32) for applying said cardiopulmonary resuscitation in response to the sensing module (40) detecting that the passenger is suffering a cardiorespiratory arrest.

## Description

### FIELD OF THE INVENTION

The present invention is in the field of car safety. In particular, the present invention relates to a system for automatization of life-preserving measures in a car for a passenger suffering a cardiorespiratory arrest.

### BACKGROUND OF THE INVENTION

A cardiorespiratory arrest is a sudden stop in effective blood circulation due to failure of the heart to pump blood effectively, which is commonly caused by a heart attack, sudden cardiac death or a coronary artery disease. When a person suffers a cardiorespiratory arrest, severe cerebral injuries or death can only be avoided by applying recovery treatment immediately. Already after three minutes of blood circulation stop, lack of oxygen in the brain can lead to irreversible damage. Therefore, any means aimed at providing quick treatment reaction to a person suffering a cardiorespiratory arrest are advantageous and worth pursuing.

The most important types of recovery treatment applicable to a person suffering a cardiorespiratory arrest are cardiopulmonary resuscitation and defibrillation. Cardiopulmonary resuscitation (CPR) typically comprises a series of chest compressions aimed at artificially restoring blood circulation in the body. Ideally, chest compressions should be applied at a rate of 100 times per minute according to current medical standards. Studies have shown that immediate CPR followed by defibrillation within 3 to 5 minutes of cardiorespiratory arrest dramatically improves the chances of survival. Defibrillation consists in delivering a therapeutic dose of electrical current to the heart of the person suffering a cardiorespiratory arrest so as to terminate a dysrhythmia and allow normal cardiac rhythm to be reestablished.

Unfortunately, many persons spend a significant amount of their time in premises or vehicles in which a quick response to a cardiorespiratory arrest cannot be guaranteed. This is the reason why continuous efforts are being made both at a technical and at an administrative level to extend the access to means allowing the application of life-preserving measures, like automatic external defibrillators or laryngeal masks. For example, numerous city councils are promoting the installation of such means in public places, such as subway and bus stations or open air spaces.

However, private vehicles have not yet been subject to such efforts. In the industrialized world, an average person spends around 275 hours per year inside a private car (American Driving Survey: Methodology and Year 1 Results, May 2013 - May 2014, AAA Foundation for Traffic Safety). A significant amount of this time is further spent in situations of traffic congestion, which may increase the risk of suffering cardiac failure.

In view of the above, there is room for technical improvements in the field of car safety and medical reanimation for passengers suffering a cardiorespiratory arrest while being inside a car.

### SUMMARY OF THE INVENTION

The problem underlying the invention is to provide a system for automatically applying life-preserving measures to a passenger in a car suffering a cardiorespiratory arrest. This problem is solved by a system according to claim 1. Alternative embodiments of the invention are described in the dependent claims.

The system of the invention comprises a sensing module, a driving module, and a control unit. The control unit is operatively connected to the sensing module and the driving module. The sensing module is configured for receiving sensing signals from one or more sensors, said sensors configured for obtaining said sensing signals related to vital signs of the passenger, said sensing signals allowing the sensing module to detect when the passenger is suffering a cardiorespiratory arrest. "Vital signs" are understood herein to have a broad meaning and to refer to any measurable signals usable to determine the health state of a person in general, and in particular to determine when a person is suffering a cardiorespiratory arrest. Vital signs may refer to any of body temperature, transpiration, oxygen saturation, blood pressure, heart rate and breathing rate. Other parameters obtainable in relation to the health state of the passenger may as well be used for these purposes. The sensing module may be configured to establish that the passenger is suffering a cardiorespiratory arrest when one or more of said vital signs reach a predetermined range or characteristic pattern. The invention will be described with general reference to cardiorespiratory arrest, which may refer in particular to a heart attack or a sudden cardiac arrest.

The driving module is configured for driving a tensioning movement of a seat belt tensioning mechanism so as to apply cardiopulmonary resuscitation (CPR) to a passenger secured by the seat belt by compressing his/her chest. A seat belt is understood herein to refer to the common kind of seat belt extensively installed in vehicles to secure the passenger against harmful movements that may result during a collision or a sudden deceleration or stop of the vehicle. In particular, a seat belt may refer to a three-point seat belt or to any kind of seat belt comprising a strap extending across the passenger's chest. A tensioning movement refers herein to a pulling movement of the seat belt tensioning mechanism resulting in a shortening and/or a tensioning of the seat belt. When applied with sufficient strength, such a tensioning movement results in a compression of the chest of the passenger in a manner that emulates the pressure exerted on the thoracic region during CPR to restore spontaneous blood circulation in a person who is in cardiac arrest. Note that the term "module" as used with regard to the sensing module and the driving module has a broad meaning and shall cover any hardware, software or combination thereof that allows carrying out the functionalities referred to herein.

The control unit is configured to control the driving module to operate the seat belt tensioning mechanism for applying said CPR in response to the sensing module detecting that the passenger is suffering a cardiorespiratory arrest. Hence when the sensing module determines that the health state of the passenger obtained through the sensors reveal that the passenger is suffering a cardiorespiratory arrest, the control unit reacts by controlling the driving module to operate the seat belt tensioning mechanism so that CPR is initiated.

The invention hence allows for automatically providing CPR measures in response to a passenger suffering a cardiorespiratory arrest in a car, in which case an immediate and proper CPR response treatment is critical for the survival of the passenger, who very often is alone inside the car and hence cannot readily be assisted by other people. Further the system of the invention makes use of components already present in a vast majority of commercially available cars and can hence be employed at low costs with little extra installation effort.

In a preferred embodiment of the invention, the driving module is configured to periodically drive the tensioning movement of the seat belt tensioning mechanism, so as to apply said CPR. According to the recommended compression-only CPR technique, the driving module then drives a tensioning movement at a constant rate. Preferably, said constant rate may be regulated such that about 100 compressions per minute be delivered to the passenger suffering a cardiorespiratory arrest.

According to preferred embodiments of the invention, the system further comprises a stimulation module operatively connected to the control unit, said stimulation module configured for driving stimulation electrodes to be provided in contact with the passenger, in particular electrodes provided on the seat belt and/or on the seat. The stimulation module is configured for delivering therapeutic doses of electrical current to the passenger's heart so as to apply a defibrillation treatment to the passenger. The control unit is configured to control the stimulation module to drive the stimulation electrodes for applying said defibrillation treatment in response to the sensing module detecting that the passenger is suffering a cardiorespiratory arrest. This provides the advantage of automatizing defibrillation measures in response to a passenger suffering a cardiorespiratory arrest in a car, in which case an immediate and proper defibrillation treatment may be critical for the reestablishment of normal cardiac rhythm and hence for the survival of the passenger.

In preferred embodiments of the invention, the driving module is further configured for being connected to an air conditioning system of the car, said air-conditioning system being configured for controlling an internal temperature of the car. The control unit is configured to control the driving module to operate said air-conditioning system for reducing the body temperature of the passenger in response to the sensing module detecting that the passenger is suffering a cardiorespiratory arrest. This has the advantage of cooling down the body temperature of a passenger suffering a cardiorespiratory arrest, which is a recommended treatment measure in order to reduce the risk of cerebral damage.

According to preferred embodiments of the invention, the system further comprises one or more sensors said sensors configured for obtaining said vital signs of the passenger and for being connected to the sensing unit, wherein at least one sensor is configured for being provided at part of components of the car to get in contact with a passenger, in particular at the seat belt and/or the seat. Sensors are understood herein in a broad sense as referring to any sensing device able to detect information regarding the vital signs of the passenger and transmitting it to the sensing module. The sensors may be placed on the seat belt or on the seat so that they are in direct or indirect (e.g. through pieces of clothing) contact with the passenger's body in such a way as to measure an electrical signal related to the passenger's health state. However, this may also be done without the need for sensors placed in contact with the passenger, for example by means of cameras, radar or other telemetry devices, which may function as sensors as well. Said electrical signal may be interpreted by the sensing module or the control unit so as to obtain information about the vital signs of the passenger. Advantageously, a system equipped with such sensors may be retrofitted in a vehicle that is originally not provided with any means required for registering said vital signs.

According to preferred embodiments of the invention, the driving module is further configured for driving a hardening process of the backrest hardening mechanism so as to harden the backrest of the seat on which the passenger is sitting. The control unit is then configured to control the driving module to operate the backrest hardening mechanism for carrying out set hardening process in response to the sensing module detecting that the passenger is suffering a cardiorespiratory arrest. Thereby, the compressing function of the seat belt during the CPR chest compressions can be eased by providing a hard surface to hold the body of the passenger during the compressions.

In a preferred embodiment of the invention, the sensing signals allow the sensing module to detect when the passenger, after suffering a cardiorespiratory arrest, has recovered from a cardiorespiratory arrest. In particular, the sensing module may be configured for recognizing when any of the vital signs registered by the sensing module, like for example blood pressure, transpiration, oxygen saturation, body temperature, heart rate, breathing rate, or a combination thereof, return to a value range corresponding to a healthy state of the passenger, and in particular a state not corresponding to a cardiorespiratory arrest. This allows limiting the duration of life-preserving measures to the strictly required duration so as to avoid possible injuries as a result of overtreatment. Further, the ability of the sensing module to recognize when the passenger has recovered from a state of cardiorespiratory arrest allows subsequent treatment measures to be initiated.

According to preferred embodiments of the invention, the driving module is further configured for driving a seat mechanism configured for adjusting the position of a seat on which the passenger is sitting, so that the body of the passenger takes a total or partial lying position, or an upright position. A seat mechanism refers herein to any mechanism suitable to modify the positioning of the seat through mechanical means, electronic means, pneumatic means or a combination thereof. The control unit may be configured to control the driving module to operate the seat mechanism to adjust the position of the seat on which the passenger is sitting to an upright position in response to the sensing module detecting that the passenger is suffering a cardiorespiratory arrest. Thereby, the position of the body of the passenger can be optimally adjusted in view of the forthcoming chest compressions carried out by means of the seat belt. The control unit may further be configured to control the driving module to operate the seat mechanism to adjust the position of the seat on which the passenger is sitting in response to the sensing module detecting that the passenger has recovered from a cardiorespiratory arrest. This way, the brain is lowered with respect to the heart of the passenger, which contributes to the reestablishment of normal blood flow after the passenger has recovered from a state of cardiorespiratory arrest,.

In a preferred embodiment of the invention, the driving module is further configured for driving a headrest mechanism configured for adjusting the position of a headrest of the seat on which the passenger is sitting, so that the head of the passenger is reclined. The control unit is then configured to control the driving module to operate the headrest mechanism to adjust the position of the headrest of the seat on which the passenger is sitting in response to the sensing module detecting that the passenger has recovered from a cardiorespiratory arrest. Advantageously, this results in the head of the passenger being tilted backwards and hence in the clearance of the airways of the passenger. This is a recommended measure for a person that has recovered from a state of cardiorespiratory arrest in order to recover normal breathing function.

According to a preferred embodiment of the invention, the system further comprises a telecommunication module configured for establishing communication with a remote device to signal to the remote device that the passenger requires emergency assistance, wherein the control unit is operatively connected to the telecommunication module and configured to control the telecommunication module to signal to the remote device that the passenger requires emergency assistance in response to the sensing module detecting that the passenger is suffering a cardiorespiratory arrest. A telecommunication module is understood herein in a broad sense to refer to any device able of establishing distance communication with a remote device for the purpose of signalling the need of emergency assistance. In particular, said telecommunication module may establish communication with a remote device by means of satellite communication, an Internet connection, radio signals, or a telephone network. This way emergency assistance for a passenger suffering a cardiorespiratory arrest can be rapidly and automatically requested, a process for which promptness is a critical factor for the survival chances of the passenger.

In preferred embodiments of the invention, the telecommunication module is configured for establishing communication with a telecommunication terminal carried by the passenger and for establishing communication with a remote device to signal that the passenger requires emergency assistance by means of said telecommunication terminal. A telecommunication terminal is understood herein to refer to any portable device able of establishing communication remotely, in particular to a cell phone. This way the system of the invention may resort to the communication capability of a telecommunication terminal carried by the passenger, for example when the vehicle is not provided with equivalent communication equipment, or when said equivalent communication equipment fails to operate properly. Further, the use of the telecommunication terminal may include use of a mobile phone network, which may be helpful for establishing the location of the car.

Preferably, the telecommunication module is further configured for signalling to the remote device a location of the car. For this purpose, the telecommunication module may by itself or assisted by the control unit exploit localization means present in the vehicle or in a telecommunication terminal carried by the passenger, in particular a GNSS navigation system or a cell phone. As a result of this the process of requesting emergency assistance for a passenger suffering a cardiorespiratory arrest is expedited inasmuch as the location of the passenger can be automatically communicated to the emergency services.

In a preferred embodiment of the invention, the control unit is further configured for being coupled for communication with illumination means and/or acoustic warning means of the car and configured to control said illumination means to emit light signals and/or to control said acoustic warning means to emit acoustic signals in response to the sensing module detecting that the passenger is suffering a cardiorespiratory arrest. For example, the control unit maycontrol the car lights and/or blinkers to emit light signals and the car horn to emit acoustic signals in response to the sensing module detecting that the passenger is suffering a cardiorespiratory arrest. This way, the emergency situation can be signaled to the exterior of the car, for instance to other car drivers or passengers and/or to pedestrians, even in cases in which the telecommunication module is unable to establish communication with a remote device.

In a preferred embodiment of the invention, the sensing module is configured for receiving signals from one or more wearable devices worn by the passenger, said wearable devices configured for obtaining sensing signals related to vital signs of the passenger, said sensing signals allowing the sensing module to detect when the passenger is suffering a cardiorespiratory arrest and/or when the passenger has recovered from a cardiorespiratory arrest. Examples of such wearable devices are wristbands, breast belts, ear clips or so-called "smart textiles" equipped with suitable sensors for detecting vital signs of the passenger, such as body temperature, transpiration, oxygen saturation, blood pressure, heart rate, breathing rate, or any combination thereof. In particular, using smart textiles, a high-quality electrocardiogram of the passenger can continuously be recorded and taken into account for determining whether the passenger is suffering a cardiorespiratory arrest.

According to preferred embodiments of the invention, the control unit is further configured for being coupled for communication with a control device of the car and for receiving information with respect to one or more driving parameters of the car from said control device, said driving parameters allowing the control unit to determine a driving condition of the car and/or to set it, in particular the current velocity of the car. A car system is understood herein in a broad sense to refer to any system, device or combination of devices able to provide the control unit with information about driving conditions of the car and able to set driving conditions when controlled to do so by the control unit. The control unit is then configured to control the operation of the driving module depending on said driving parameters. In particular, the control unit might be configured to operate the driving module in response to the sensing module detecting that the passenger is suffering a cardiorespiratory arrest or not to do so depending on the driving parameters. This has the advantage of providing to the control unit information about the current state of the car, which may be beneficial in order to avoid the initiation of life-preserving measures when this can jeopardize the safety of the passenger and/or other occupants of the car. For example, the control unit may be configured for registering the velocity of the car and to control the driving module not to operate in response to the sensing module detecting that the passenger is suffering a cardiorespiratory arrest when the velocity of the car is beyond a predetermined velocity.

Preferably, the car system allows the control unit to detect a situation in which the passenger no longer requires the life-preserving measures because he/she is already being assisted by emergency services or a third person or e.g. because the passenger is able to leave the car. For this purpose, the control unit may be configured for detecting when a door - preferably the door of the car corresponding to the seat on which the passenger is sitting - is opened or manipulated.

In a preferred embodiment of the invention, the control unit is further configured for being coupled for communication with a control device of the car and for controlling driving conditions of the car, in particular the velocity of the car, wherein the control unit is configured for controlling the car to stop in response to the sensing module detecting that the passenger is suffering a cardiorespiratory arrest. This way, the control unit has the ability of bringing the car to a stop when the passenger is suffering a cardiorespiratory arrest and is not able to control the car.

A number of life-preserving measures have been disclosed herein which might be initiated in response to the sensing module detecting that the passenger is suffering a cardiorespiratory arrest. Said life-preserving measures may be applied in any order, preferably attending to corresponding medical recommendations and official regulations.

### BRIEF DESCRIPTION OF THE DRAWINGS

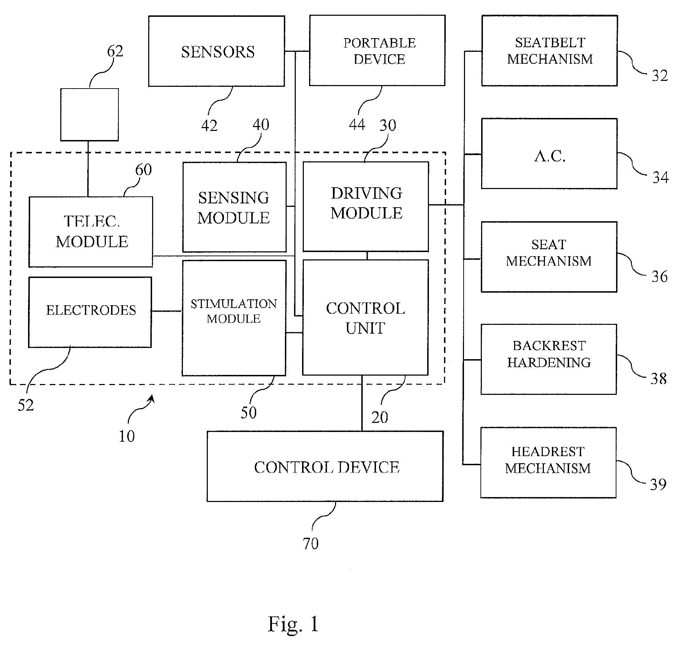

- Fig. 1: is a schematic representation of a system according to an embodiment of the invention.

### DESCRIPTION OF PREFERRED EMBODIMENTS

For the purposes of promoting an understanding of the principles of the invention, reference will now be made to a preferred embodiment illustrated in the drawings, and specific language will be used to describe the same. It will nevertheless be understood that no limitation of the scope of the invention is thereby intended, such alterations and further modifications in the illustrated apparatus and such further applications of the principles of the invention as illustrated therein being contemplated as would normally occur now or in the future to one skilled in the art to which the invention relates. Exemplary embodiments of the invention will be described assuming that the passenger that is suffering a cardiorespiratory arrest is the driver of the car. However, the invention may as well be adapted for applying life-preserving measures to any other passenger of a car.

Figure 1 shows a system 10 for automatically applying life-preserving measures to a passenger in a car suffering a cardiorespiratory arrest according to an embodiment of the invention. The system 10 comprises a sensing module 40 configured for receiving sensing signals from a plurality of sensors 42 and from a wearable device 44 worn by the passenger, and for obtaining sensing signals related to vital signs of the passenger obtained from the sensing signals. The sensing signals allow the sensing module 40 to detect when the passenger is suffering a cardiorespiratory arrest. The sensors 42 are attached at parts of the car in contact with the passenger, like the seat belt, the seat and the steering wheel. In the embodiment shown, the sensors 42 are already installed in the vehicle and hence not considered to be part of the system 10. However, the sensors 42 may be comprised in the system 10 The wearable device 44 can be a wristband, a watch, an ear clip, a breast belt, or a smart textile configured for obtaining vital signs of the passenger. The sensors may also be provided in an implantable device, such as a pacemaker.

The system 10 further comprises a control unit 20, a stimulation module 50, and a telecommunication module 60. The control unit 20 is operatively connected to the driving module 30, the sensing module 40, the stimulation module 50, and the telecommunication module 60.

The driving module 30 is configured for driving a tensioning movement of a seat belt tensioning mechanism 32 so as to apply cardiopulmonary resuscitation to a passenger secured by the seat belt by compressing the passenger's chest. Further, the driving module 30 is operationally connected to an air conditioning system 34, a seat mechanism 36, a backrest hardening mechanism 38, and a headrest mechanism 39 of the car. The air conditioning system 34 is configured for controlling an internal temperature of the car. The seat mechanism 36 is configured for adjusting the position of a seat on which the passenger is sitting. The backrest hardening mechanism 38 is configured for hardening the backrest of the seat on which the passenger is sitting. The headrest mechanism 39 is configured for adjusting the position of a headrest of the seat on which the passenger is sitting.

The telecommunication module 60 is configured for establishing communication with a remote device to signal to the remote device that the passenger requires emergency assistance. For this purpose, the telecommunication module 60 can establish communication with a telecommunication terminal 62 carried by the passenger, like a mobile phone or a similar device.

When the passenger suffers a cardiorespiratory arrest, the corresponding symptomatic state is reflected by the vital signs of the passenger, which are registered by the sensors 42 and the wearable device(s) 44. The sensing module 40 detects that the passenger is having a cardiorespiratory arrest and notifies the situation to the control unit 20.

In response to the sensing module 40 detecting that the passenger is suffering a cardiorespiratory arrest, the control unit 20 controls the telecommunication module 60 using the telecommunication terminal 62 to send to a remote device a signal to notify that the passenger requires emergency assistance. The telecommunication module 60 further uses the telecommunication terminal 62 to signal to the remote device the location of the car, wherein the location of the car can be obtained from a navigation system of the car or from the telecommunication terminal 62 itself.

Further, in response to the sensing module 40 detecting that the passenger is suffering a cardiorespiratory arrest, the control unit 20 controls the driving module 30 to operate the seat mechanism 36 to modify the position of the seat on which the passenger is sitting such that the body of the passenger takes an upright position. Additionally, the control unit 20 controls the driving module 30 to operate the backrest hardening mechanism 38 to harden the backrest of the seat on which the passenger is sitting and to operate the air conditioning system 34 for reducing the inner temperature of the car so as to reduce the body temperature of the passenger as well. Finally, the control unit 20 controls the driving module 30 to operate the seat belt tensioning mechanism 32 for applying cardiopulmonary resuscitation to the passenger. For this purpose, the driving module 30 drives a periodic tensioning movement of the seat belt through the seat belt mechanism 32 so as to apply said cardiopulmonary resuscitation.

The system 10 further comprises a set of stimulation electrodes 52 provided in contact with the passenger on the seat belt, the seat and/or the steering wheel. The stimulation module 50 is configured for driving the stimulation electrodes 52 for delivering therapeutic doses of electrical current to the passenger's heart so as to apply a defibrillation treatment to the passenger. In a further response to the sensing module 40 detecting that the passenger is suffering a cardiorespiratory arrest, the control unit 20 controls the stimulation module 50 to drive the stimulation electrodes 52 for applying said defibrillation treatment.

The control unit 20 is further operatively connected to a control device 70 of the car, which allows the control unit 20 to register driving parameters of the car that define the driving conditions, like the velocity, braking distance and the distance to other cars, and to actively set them. The control unit 20 is configured to control the driving module 30 not to operate in response to the sensing module detecting that the passenger is suffering a cardiorespiratory arrest when the driving conditions inferred from the driving parameters reflect that initiating the life-preserving measures could be a potential source of risk. For example, the control unit 20 disables the driving module 30 when the velocity of the car exceeds a predefined velocity limit.

In addition, the control unit 20 is configured for controlling driving conditions of the car, in particular its velocity. This way, when the sensing module 40 detects that the passenger is suffering a cardiorespiratory arrest, the control unit 20 can reduce the velocity of the car until it stops. In a preferred embodiment, the control unit 20 causes the control device 70 of the car to switch to an autonomous driving mode and to carry out a controlled emergency stop of the car.

The sensing signals obtained from the sensors 42 and the wearable device(s) 44 further allow the sensing module 40 to detect when the passenger, after suffering a cardiorespiratory arrest, has recovered from it. When this happens, the control unit 20 controls the driving module 30 to operate the seat mechanism 36 to modify the position of the seat on which the passenger is sitting such that the body of the passenger takes a total or a partial lying position. Further, the control unit 20 controls the driving module 30 to operate the headrest mechanism 39 to modify the position of a headrest of the seat on which the passenger is sitting so that the head of the passenger is reclined.

Although preferred exemplary embodiments are shown and specified in detail in the drawings and the preceding specification, these should be viewed as purely exemplary and not as limiting the invention. It is noted in this regard that only the preferred exemplary embodiments are shown and specified, and all variations and modifications should be protected that presently or in the future lie within the scope of protection of the invention as defined in the claims.

### REFERENCE SIGN LIST

- 10: system of the invention
- 20: control unit
- 30: driving module
- 32: seat belt tensioning mechanism
- 34: air conditioning system
- 36: seat mechanism
- 38: backrest hardening mechanism
- 39: headrest mechanism
- 40: sensing module
- 42: sensors
- 44: wearable device
- 50: stimulation module
- 52: stimulation electrodes
- 60: telecommunication module
- 62: telecommunication terminal
- 70: control device of the car

## Claims

1. A system (10) for automatically applying life-preserving measures to a passenger in a car suffering a cardiorespiratory arrest, wherein the system comprises:
a sensing module (40) configured for receiving sensing signals from one or more sensors (42), said sensors (42) configured for obtaining said sensing signals related to vital signs of the passenger, said sensing signals allowing the sensing module (40) to detect when the passenger is suffering a cardiorespiratory arrest;
a driving module (30) configured for driving a tensioning movement of a seat belt tensioning mechanism (32) so as to apply cardiopulmonary resuscitation to a passenger secured by the seat belt by compressing his/her chest; and
a control unit (20) operatively connected to the sensing module (40) and the driving module (30);
wherein the control unit (20) is configured to control the driving module (30) to operate the seat belt tensioning mechanism (32) for applying said cardiopulmonary resuscitation in response to the sensing module (40) detecting that the passenger is suffering a cardiorespiratory arrest.

2. The system (10) of claim 1, wherein the driving module (30) is configured to periodically drive the tensioning movement of the seat belt tensioning mechanism (32) so as to apply said cardiopulmonary resuscitation.

3. The system (10) of claim 1 or 2, wherein the system (10) further comprises a stimulation module (50) operatively connected to the control unit (20), said stimulation module (50) configured for driving stimulation electrodes (52) to be provided in contact with the passenger, in particular electrodes (52) provided on the seat belt and/or on the seat, and for delivering therapeutic doses of electrical current to the passenger's heart so as to apply a defibrillation treatment to the passenger;
wherein the control unit (20) is configured to control the stimulation module (50) to drive the stimulation electrodes (52) for applying said defibrillation treatment in response to the sensing module (50) detecting that the passenger is suffering a cardiorespiratory arrest.

4. The system (10) of any of the preceding claims, wherein the driving module (30) is further configured for being connected to an air-conditioning system of the car (34), said air-conditioning system (34) configured for controlling an internal temperature of the car; wherein the control unit (20) is configured to control the driving module (30) to operate said air-conditioning system (34) for reducing the body temperature of the passenger in response to the sensing module (40) detecting that the passenger is suffering a cardiorespiratory arrest.

5. The system (10) of any of the preceding claims, wherein the system (10) further comprises one or more sensors (42), said sensors (42) configured for obtaining said vital signs of the passenger and for being connected to the sensing module (40), wherein at least one sensor (42) is configured for being provided at parts or components of the car to get in contact with a passenger, in particular at the seat belt and/or the seat.

6. The system (10) of any of the preceding claims, wherein the driving module (30) is further configured for driving a seat mechanism (36) configured for adjusting the position of a seat on which the passenger is sitting, so that the body of the passenger takes an upright position;
wherein the control unit (20) is configured to control the driving module (30) to operate the seat mechanism (36) to adjust the position of the seat on which the passenger is sitting in response to the sensing module (40) detecting that the passenger is suffering a cardiorespiratory arrest.

7. The system (10) of any of the preceding claims, wherein the driving module (30) is further configured for driving a hardening process of a backrest hardening mechanism (38) so as to harden the backrest of the seat on which the passenger is sitting;
wherein the control unit (20) is configured to control the driving module (30) to operate the backrest hardening mechanism (38) for carrying out said hardening process in response to the sensing module (40) detecting that the passenger is suffering a cardiorespiratory arrest.

8. The system (10) of any of the preceding claims, wherein said sensing signals allow the sensing module (40) to detect when the passenger, after suffering a cardiorespiratory arrest, has recovered from a cardiorespiratory arrest.

9. The system (10) of claim 8, wherein the driving module (30) is further configured for driving a seat mechanism (36) configured for adjusting the position of a seat on which the passenger is sitting, so that the body of the passenger takes a total or partial lying position;
wherein the control unit (20) is configured to control the driving module (30) to operate the seat mechanism (36) to adjust the position of the seat on which the passenger is sitting in response to the sensing module (40) detecting that the passenger has recovered from a cardiorespiratory arrest.

10. The system (10) of claim 8 or 9, wherein the driving module (30) is further configured for driving a headrest mechanism (39) configured for adjusting the position of a headrest of the seat on which the passenger is sitting, so that the head of the passenger is reclined; wherein the control unit (20) is configured for controlling the driving module (30) to operate the headrest mechanism (39) to adjust the position of the headrest of the seat on which the passenger is sitting in response to the sensing module (40) detecting that the passenger has recovered from a cardiorespiratory arrest.

11. The system (10) of any of the preceding claims, wherein the system (10) further comprises a telecommunication module (60) configured for establishing communication with a remote device to signal to the remote device that the passenger requires emergency assistance;
wherein the control unit (20) is operatively connected to the telecommunication module (60) and configured to control the telecommunication module (60) to signal to the remote device that the passenger requires emergency assistance in response to the sensing module (40) detecting that the passenger is suffering a cardiorespiratory arrest; and wherein the telecommunication module (60) is preferably configured for establishing communication with a telecommunication terminal (62) carried by the passenger and for establishing communication with a remote device to signal that the passenger requires emergency assistance by means of said telecommunication terminal (62).

12. The system (10) of claim 11, wherein the telecommunication module (62) is further configured for signalling to the remote device a location of the car.

13. The system (10) of any of the preceding claims, wherein the control unit (20) is further configured for being coupled for communication with illumination means and/or acoustic warning means of the car and configured to control said illumination means to emit light signals and/or to control said acoustic warning means to emit acoustic signals in response to the sensing module (40) detecting that the passenger is suffering a cardiorespiratory arrest.

14. The system (10) of any of the preceding claims, wherein the a sensing module (40) is configured for receiving sensing signals from one or more wearable devices (44) worn by the passenger, said wearable devices (44) configured for obtaining sensing signals related to vital signs of the passenger, said sensing signals allowing the sensing module (40) to detect when the passenger is suffering a cardiorespiratory arrest and/or when the passenger has recovered from a cardiorespiratory arrest.

15. The system (10) of any of the preceding claims, wherein the control unit (20) is further configured for being coupled for communication with a control device (70) of the car and for receiving information with respect to one or more driving parameters of the car from said control device (70), said driving parameters allowing the control unit (20) to determine a driving condition of the car and/or to set it, in particular the current velocity of the car, and wherein the control unit (20) is configured to control the operation of the driving module (30) depending on said driving parameters; and/or
wherein the control unit (20) is further configured for being coupled for communication with a control device (70) of the car and for controlling driving conditions of the car, in particular the velocity of the car, wherein the control unit (20) is configured to control the car to stop in response to the sensing module (40) detecting that the passenger is suffering a cardiorespiratory arrest.
